(19) Europäisches Patentamt
European Patent Office
Office européen des brevets

(11) EP 3 027 586 B1

(12) FASCICULE DE BREVET EUROPEEN

(45) Date de publication et mention de la délivrance du brevet:
**26.02.2020 Bulletin 2020/09**

(21) Numéro de dépôt: **14750583.8**

(22) Date de dépôt: **24.07.2014**

(51) Int Cl.:
***C07C 67/08*** (2006.01) ***C07C 67/327*** (2006.01)
***C07C 51/09*** (2006.01) ***C07C 69/54*** (2006.01)
***C07C 57/04*** (2006.01)

(86) Numéro de dépôt international:
**PCT/FR2014/051928**

(87) Numéro de publication internationale:
**WO 2015/015100 (05.02.2015 Gazette 2015/05)**

# (54) PROCÉDÉ DE PRODUCTION EN CONTINU D'ACRYLATES LÉGERS PAR ESTÉRIFICATION D'UN ACIDE ACRYLIQUE DE GRADE ESTER BRUT

VERFAHREN ZUR KONTINUIERLICHEN HERSTELLUNG VON LEICHTEN ACRYLATEN DURCH VERESTERUNG EINER ROHEN ESTERGRAD-ACRYLSÄURE

METHOD FOR CONTINUOUS PRODUCTION OF LIGHT ACRYLATES BY ESTERIFICATION OF A RAW ESTER-GRADE ACRYLIC ACID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.07.2013 FR 1357447**

(43) Date de publication de la demande:
**08.06.2016 Bulletin 2016/23**

(73) Titulaire: **Arkema France**
**92700 Colombes (FR)**

(72) Inventeurs:
- **FAUCONET, Michel**
  **57730 Valmont (FR)**
- **ROUNDY, Roger**
  **Rosharon, Texas 77583 (US)**
- **DENIS, Stephane**
  **57660 Leyviller (FR)**
- **DANIEL, Samuel**
  **Malvern, Pennsylvania 19355 (US)**

(74) Mandataire: **Albani, Dalila et al**
**ARKEMA France**
**Département Propriété Industrielle**
**420, rue d'Estienne d'Orves**
**92705 Colombes Cedex (FR)**

(56) Documents cités:
**WO-A1-98/52903**
**WO-A1-98/52904**
**CA-A1- 2 193 407**
**US-A- 4 076 950**
**US-A1- 2007 280 866**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 027 586 B1

## Description

### Domaine technique

La présente invention concerne la fabrication d'acrylates légers, et a notamment pour objet un procédé de préparation en continu d'acrylates légers par estérification directe à partir d'un flux d'acide acrylique brut et d'un alcool léger, en particulier de méthanol ou d'éthanol.

### Art antérieur et problème technique

Les esters acryliques légers (ou acrylates légers) sont préparés à partir d'acide acrylique et d'alcool léger (tel que méthanol ou éthanol), par simple estérification. La gamme d'utilisations pour la fabrication de polymères est étendue. L'acrylate de méthyle (AM) est très souvent utilisé dans les procédés de copolymérisation pour fabriquer des fibres. L'acrylate d'éthyle (AE) est en particulier utilisé dans les procédés de copolymérisation pour conférer la cohésion de fibres textiles.

L'obtention de ces monomères à un degré de pureté satisfaisant pour l'application industrielle finale est donc essentielle et souvent délicate, et requiert des techniques de purification onéreuses. Ainsi, pour faire face à ces contraintes de pureté, une attention toute particulière est portée à la qualité de l'acide acrylique qui doit être exempte d'impuretés qui pourraient générer des sous-produits dans l'acrylate léger. C'est pourquoi, la réaction d'estérification est de façon générale réalisée à partir d'un acide acrylique purifié.

Pour la synthèse de l'acide acrylique (AA), la voie industrielle la plus largement utilisée est celle de l'oxydation du propylène. Cette synthèse de l'acide acrylique est appelée synthèse pétrochimique et comporte deux étapes, la première vise l'oxydation du propylène en acroléine avec production simultanée d'une molécule d'eau par molécule d'acroléine et la seconde l'oxydation de l'acroléine en acide acrylique. Cette synthèse est généralement conduite dans deux réacteurs utilisant deux systèmes catalytiques spécifiques de chacune des étapes d'oxydation, ou dans un seul réacteur comportant deux lits de catalyseur distincts.

Une autre voie de synthèse de l'acide acrylique utilise le glycérol ou la glycérine comme matière première, qui est soumis tout d'abord à une déshydratation conduisant à l'acroléine avec production simultanée de 2 molécules d'eau par molécule d'acroléine, cette dernière étant ensuite soumise à une oxydation pour former l'acide acrylique. Cette voie comporte aussi deux étapes avec comme point commun la présence de l'acroléine comme produit intermédiaire. Elle est en cours de développement industriel car il s'agit là d'un procédé « vert » utilisant une matière première naturelle renouvelable et non plus fossile comme le propylène.

L'effluent sortant du réacteur après l'oxydation de l'acroléine en acide acrylique contient outre les produits de réaction, acide acrylique et eau, toute une série de sous-produits constituant des impuretés et cela aussi bien par la voie propylène que par la voie glycérol. Ces sous-produits sont notamment :

- des composés légers incondensables dans les conditions de température et de pression habituellement mises en œuvre (azote, oxygène, réactif résiduel, monoxyde et dioxyde de carbone formés en faible quantité par oxydation ultime) ;
- des composés légers condensables : en particulier l'eau, acroléine non convertie, des aldéhydes légers, comme le formaldéhyde, l'acétaldéhyde et l'acide acétique, principale impureté générée dans la section réaction ;
- des composés lourds : notamment furfuraldéhyde, benzaldéhyde, anhydride maléique, acide benzoïque.

Dans le procédé de synthèse de l'acide acrylique, l'effluent sortant du réacteur, quelle que soit la voie choisie, est soumis à une chaîne de traitement et de purification que l'on peut résumer comme suit :
L'acide acrylique est récupéré à partir du mélange gazeux issu de la 2ème étape, en introduisant ce gaz en pied d'une colonne d'absorption où il rencontre à contre-courant un solvant introduit en tête de colonne. Dans la plupart des procédés décrits, le solvant mis en œuvre dans cette colonne est l'eau ou un solvant hydrophobe à haut point d'ébullition. Cette étape permet par élimination des incondensables la formation d'acide acrylique « brut ».

Dans le cas de procédés d'absorption utilisant l'eau comme solvant absorbant, les étapes de purification complémentaires comprennent une étape de déshydratation, généralement réalisée en présence de solvant non miscible à l'eau, dans une colonne d'extraction ou de distillation hétéroazéotropique, puis une étape d'élimination des composés légers, en particulier l'acide acétique et l'acide formique, cette étape étant généralement dénommée « étêtage ». Enfin, une étape de séparation des composés lourds est réalisée par distillation, cette étape étant généralement dénommée « équeutage » et conduisant à un acide acrylique « technique ». Dans le cas de procédés utilisant un solvant hydrophobe, les étapes sont essentiellement les mêmes, sauf l'élimination de l'eau qui est réalisée en tête de la première colonne d'absorption.

Récemment, de nouvelles technologies « sans solvant » de récupération/purification d'acide acrylique sont

apparues, impliquant un nombre réduit d'étapes de purification et supprimant l'introduction de solvant organique externe. Le brevet EP 2 066 613 décrit un procédé de récupération d'acide acrylique sans utiliser de solvant azéotropique et ne mettant en œuvre que deux colonnes de purification du mélange réactionnel gazeux refroidi : a) une colonne de déshydratation où le flux gazeux distillé en tête est condensé et renvoyé à la colonne de déshydratation sous forme de reflux pour absorber l'acide acrylique, b) et une colonne de finition alimentée par le flux de pied de la première colonne, dans laquelle, i) l'eau et l'acide acétique résiduels sont distillés en tête et recyclés en pied de la première colonne, ii) un flux comprenant les sous-produits lourds et de l'acide acrylique est éliminé en pied pour être utilisé éventuellement pour la production d'esters acryliques, et iii) un flux d'acide acrylique de grade technique est récupéré par soutirage latéral sous forme de liquide ou de vapeur.

Au cours de ces différentes étapes de traitement/purification, peuvent se produire des réactions secondaires covalentes d'addition de Michael sur la double-liaison de l'acide acrylique, générant des composés répondant au terme général d'adduits de Michael.

Il s'agit essentiellement d'oligomères de l'acide acrylique, principalement composés de molécules adduits de Michael appelés acide acrylique dimère (acide 3-acryloxypropionique, n=1) et acide acrylique trimère (acide 3-acryloxy, 3-propioxy propionique, n=2). Ils sont caractérisés par un point d'ébullition supérieur au point d'ébullition des produits mis en œuvre dans la réaction, et vont se retrouver dans la fraction de composés lourds séparée au cours de l'étape finale d'équeutage.

$$n{+}1\ H_2C{=}CH{-}C(O)OH \longrightarrow H_2C{=}CH{-}C(O)O{-}\left(CH_2{-}CH_2{-}C(O)O\right)_n{-}H$$

D'autres adduits de Michael peuvent également coexister avec les dérivés d'addition de l'acide acrylique sur lui-même, qui mettent en jeu l'addition nucléophile de composés présents dans le milieu de purification de l'acide acrylique, comme par exemple l'eau, sur la double liaison de l'acide acrylique ou d'oligomères d'acide acrylique :

$$H_2O + n\ H_2C{=}CH{-}C(O)OH \longrightarrow HO{-}\left(H_2C{-}CH_2{-}C(O)O\right)_n{-}H$$

Le processus de traitement/purification de l'acide acrylique conduit donc à l'obtention, d'une part d'un flux d'acide acrylique purifié généralement dénommé de grade « technique », utilisé notamment comme matière première pour la production d'acrylates légers, d'autre part d'un flux comportant des composés lourds dont notamment des oligomères d'acide acrylique en quantité importante.

Dans le procédé traditionnel, la fraction lourde est éliminée, mais le plus souvent afin d'éviter une perte de produits valorisables, elle est soumise à un traitement thermique à haute température, en présence ou non d'un catalyseur, pour dissocier les oligomères et récupérer l'acide acrylique monomère, le résidu ultime étant ensuite éliminé et l'acide acrylique récupéré étant recyclé comme matière première dans un procédé d'estérification (EP 887 334 ; FR 1 351 243). Les inconvénients de ce type de traitement sont la viscosité importante du résidu qui devient non véhiculable dans les conduites, et l'encrassement de la paroi du réacteur de craquage.

Par ailleurs, la production d'esters acryliques comprenant la réaction d'estérification d'acide acrylique technique avec un alcool, suivie d'un traitement de purification, s'accompagne elle aussi de la formation d'adduits de Michael : il s'agit principalement des oligomères d'acide acrylique et des dérivés issus de leur estérification avec l'alcool utilisé pour la réaction d'estérification, ainsi que des produits d'addition de Michael de l'alcool sur les doubles liaisons des composés cités :

$$H_2C{=}CH{-}C(O)O{-}\left(CH_2{-}CH_2{-}C(O)O\right)_n{-}H + ROH \longrightarrow H_2C{=}CH{-}C(O)O{-}\left(CH_2{-}CH_2{-}C(O)O\right)_n{-}R$$

$$H_2C{=}CH{-}C(O)O{-}\left(CH_2{-}CH_2{-}C(O)O\right)_n{-}R + ROH \longrightarrow RO{-}H_2C{-}CH_2{-}C(O)O{-}\left(CH_2{-}CH_2{-}C(O)O\right)_n{-}R$$

Dans le document US 3,868,410, ces adduits de Michael sont avantageusement convertis en monomères par traitement thermique à une température supérieure à 180°C, en vue de leur recyclage à l'étape d'estérification.

Afin d'améliorer le rendement de récupération des produits nobles générés par craquage thermique des fractions

lourdes formées, d'une part lors de la synthèse de l'acide acrylique, et d'autre part lors de la synthèse des esters acryliques, il a été proposé, dans le document EP 2 727 964, d'opérer la dissociation thermique sur un mélange de ces fractions lourdes, en l'absence de catalyseur, ce qui a pour effet de réduire les encrassements dans le réacteur de dissociation ainsi que la viscosité du résidu obtenu à l'issue de l'opération de dissociation thermique.

Outre le fait que les procédés précités ne résolvent pas les problèmes inhérents à la formation d'adduits de Michael au cours des étapes de synthèse et/ou purification de l'acide acrylique et/ou des esters acryliques, tels que :

- Utilisation d'inhibiteurs de polymérisation en grande quantité pour limiter les réactions radicalaires de polymérisation ;
- Complexité des étapes de séparation des fractions lourdes ;
- Coût élevé du craquage thermique des fractions lourdes pour récupérer les composés nobles ;
- Difficulté d'élimination des résidus lourds après craquage et encrassement du réacteur de craquage ;

les procédés d'estérification d'acide acrylique présentent des inconvénients qui proviennent directement de l'utilisation d'un grade d'acide acrylique purifié comme matière première pour produire les esters acryliques.

Pour réduire ces inconvénients, le procédé de production d'acrylates légers décrit dans le document WO 91/01966 utilise une solution aqueuse d'acide acrylique brut comme matière première pour la réaction d'estérification. La solution aqueuse provient de la colonne d'absorption du mélange gazeux issu de l'oxydation de l'acroléine dans le procédé de production de l'acide acrylique, et elle comprend de 50% à 70% d'acide acrylique et les impuretés inhérentes à sa production. Dans le procédé de WO 91/01966, la solution aqueuse d'acide acrylique brut n'est pas purifiée dans une installation spécifique de purification, préalablement à son introduction dans le réacteur d'estérification ; elle est introduite directement en fond de la colonne de distillation servant à séparer les produits issus du réacteur d'estérification, pour conduire à un flux enrichi en acide acrylique en pied de ladite colonne, qui est recyclé dans le réacteur d'estérification. Ce procédé n'est cependant pas économique à une échelle industrielle.

Le document WO 00/78702 décrit un procédé de synthèse d'esters acryliques, en particulier d'acrylate de butyle, dans lequel la réaction d'estérification est effectuée à une température et une pression suffisantes pour permettre le craquage des adduits de Michael formés in situ ou introduits dans le réacteur, dans une même zone de réaction, et vaporiser l'ester produit. Typiquement, la réaction est effectuée à une température allant de 100 à 160°C, et à une pression allant de 0,01 à 100 bars. Ce procédé permet l'utilisation d'acide acrylique de pureté faible, notamment d'acide acrylique comportant de l'acide acétique et/ou dimère et/ou d'autres adduits de Michael à une teneur supérieure à 0,5%.

Dans le document US 2004/0236143, le procédé de production d'esters acryliques peut être mis en œuvre à partir d'un grade d'acide acrylique brut, c'est-à-dire comportant de l'acide acétique, des aldéhydes, des composés lourds tel que anhydride maléique, ce grade d'acide acrylique brut étant éventuellement prétraité à l'aide d'un composé aminé pour réduire sa teneur en composés carbonylés, et dont la teneur en dimères est de l'ordre de 0,01 - 5%. Les étapes de purification conduisent à l'obtention d'un acrylate de méthyle de pureté supérieure à 99,9%.

Dans les documents WO 98/52903 et WO 98/52904, il a été proposé de produire par estérification directe de l'acrylate de butyle dans deux réacteurs placés en série opérant à deux températures différentes, la température du second réacteur étant plus élevée de façon à poursuivre la réaction d'estérification et dissocier thermiquement les produits lourds. Le premier réacteur est alimenté par des réactifs purs (butanol et acide acrylique) et par un flux recyclé de produits lourds, notamment une fraction d'esters lourds séparée lors de la purification de l'acrylate de butyle. Ce procédé nécessite l'utilisation de deux réacteurs et utilise de l'acide acrylique pur comme matière première.

Le document US 2007/280866 décrit la dissociation thermique d'oligomères d'acide acrylique en présence d'un réactif clivant tel que le butanol. La dissociation est réalisée en mode discontinu. Les conditions de la réaction ne sont pas adaptées pour la mise en oeuvre d'un procédé de production industrielle en continu.

Le problème que la présente invention se propose de résoudre consiste à mettre au point un procédé de fabrication d'esters acryliques légers n'ayant pas les inconvénients des procédés existants, et permettant de préparer en l'acrylate de méthyle ou l'acrylate d'éthyle de haute pureté, à partir d'un grade d'acide acrylique brut comportant une forte teneur en adduits de Michael, dénommé dans la suite de la description de l'invention « de grade ester brut ». L'objectif visé est aussi d'atteindre un haut rendement d'estérification de l'ensemble de l'acide acrylique valorisable contenu dans l'acide acrylique de grade ester brut, sans avoir recours à un dispositif spécifique de craquage desdits adduits.

Un des objectifs de la présente invention est ainsi de valoriser dans un procédé d'estérification, l'ensemble des produits nobles potentiellement récupérables dans une fraction lourde générée dans un procédé de synthèse d'acide acrylique. La présente invention a également pour objectif de réduire globalement les quantités de résidu final à incinérer dans des procédés de production d'acide acrylique et/ou d'esters acryliques, dans des conditions permettant d'obtenir le résidu final avec une viscosité acceptable pour faciliter son élimination.

Selon le procédé de l'invention, les adduits de Michael générés au cours de la réaction d'estérification, tels que des oligomères d'acide acrylique ou d'acrylate peuvent être dissociés thermiquement au sein du réacteur au fur et à mesure de leur formation, optimisant ainsi la productivité de l'installation.

Le procédé selon l'invention ne nécessite donc qu'un nombre modéré d'étapes dans un appareillage simplifié, et produit des acrylates légers de haute pureté avec un résidu ultime suffisamment fluide pour être éliminé facilement.

Résumé de l'invention

L'invention a pour objet un procédé de préparation en continu d'acrylate léger choisi parmi l'acrylate de méthyle et l'acrylate d'éthyle par réaction de l'alcool léger correspondant choisi parmi le méthanol et l'éthanol, avec un flux d'acide acrylique de grade ester brut, en présence d'au moins un catalyseur acide et d'au moins un inhibiteur de polymérisation, dans une zone réactionnelle comprenant un réacteur raccordé à une unité de distillation, caractérisé en ce que :

- le flux d'acide acrylique de grade ester brut comprend des oligomères d'acide acrylique à une teneur massique supérieure à 8% ;
- le rapport molaire d'alcool par rapport à l'acide acrylique contenu sous forme de monomère, dimère ou trimère dans le flux d'acide acrylique de grade ester brut est compris entre 1,2 et 1,5 ;
- la température du réacteur est supérieure à 130°C ;
- la concentration massique de catalyseur acide est maintenue supérieure à 2,5% dans le milieu réactionnel ;
- on maintient un excès d'acrylate léger formé dans le réacteur ;
- on ajuste la concentration en inhibiteur de polymérisation dans le réacteur à une valeur supérieure à 50 ppm ;
- l'effluent sortant de l'unité de distillation est soumis à une chaîne de traitement et de purification conduisant à l'obtention d'un acrylate léger purifié ;
- on maintient un temps de séjour du résidu de réaction dans le réacteur supérieur à 50 heures.

Le procédé de l'invention est particulièrement bien adapté pour optimiser la productivité et le bilan économique d'un procédé de fabrication d'acrylates légers.

Selon un mode de réalisation de l'invention, le flux d'acide acrylique de grade ester brut provient d'un procédé de production utilisant le propylène ou le propane comme matière première.

Selon un mode de réalisation de l'invention, le flux d'acide acrylique de grade ester brut provient d'un procédé de production utilisant le glycérol ou la glycérine comme matière première.

Selon un mode de réalisation, le flux d'acide acrylique de grade ester brut est issu d'un procédé de déshydratation de l'acide lactique ou du lactate d'ammonium en acide acrylique ou d'un procédé de déshydratation de l'acide 3-hydroxypropionique ou de son sel d'ammonium en acide acrylique.

Exposé détaillé de l'invention

L'invention est maintenant décrite plus en détail et de façon non limitative dans la description qui suit.

Dans ce qui suit, l'"acide acrylique valorisable" est constitué de l'acide acrylique monomère et des dérivés d'addition de Michael inhérents à la synthèse de l'acide acrylique, en particulier les oligomères d'acide acrylique, présents dans l'acide acrylique de grade ester brut.

Dans ce qui suit, les expressions "dissociation thermique" et "craquage thermique" ont la même signification ; l'expression "compris entre" ou "allant de" s'interprète avec bornes comprises.

Par résidu de réaction, on entend la fraction enrichie de sous-produits lourds ne réagissant pas, accumulée au sein du réacteur qu'il est nécessaire de purger périodiquement du réacteur.

Sauf indication contraire, les concentrations décrites dans l'exposé de l'invention sont des concentrations massiques.

Flux d'acide acrylique de grade ester brut

Le procédé selon lequel le flux d'acide acrylique de grade ester brut a été obtenu n'a pas d'importance pour le procédé selon l'invention, dans la mesure où il s'agit d'un flux d'acide acrylique comportant une teneur élevée en adduits de Michael, notamment une teneur massique en oligomères d'acide acrylique supérieure à 8%, en particulier une teneur en dimères d'acide acrylique supérieure à 8%, de préférence allant de 8% à 25%, et une teneur en trimères d'acide acrylique supérieure à 0,1%, de préférence allant de 0,5 à 3%.

Le flux d'acide acrylique de grade ester brut comporte généralement une teneur en acide acrylique valorisable supérieure à 90%.

Le flux d'acide acrylique de grade ester brut peut contenir en outre des sous-produits lourds à point d'ébullition élevé, inhérents à la synthèse de l'acide acrylique, tels que furfuraldéhyde, anhydride maléique, benzaldéhyde ou acide benzoïque, et des inhibiteurs de polymérisation.

La teneur massique en composés lourds peut être typiquement :

Furfuraldéhyde : 0,03 - 0,5%
Anhydride maléique : 0,3-4%
Benzaldéhyde : 0,05-0,5%
Acide benzoïque : 0,2-1%

Selon un mode de réalisation, l'acide acrylique de grade ester brut peut être obtenu au cours de la purification d'acide acrylique brut récupéré à l'aide d'une colonne d'absorption alimenté en un solvant, tel que l'eau ou un solvant hydrophobe, en sortie du réacteur de synthèse de l'acide acrylique.

Cette purification peut notamment comprendre une première étape de déshydratation, généralement réalisée en présence de solvant non miscible à l'eau, dans une colonne d'extraction ou de distillation hétéroazéotropique, suivie d'une étape d'élimination des composés légers, en particulier l'acide acétique et l'acide formique, cette étape étant généralement dénommée « étêtage ». Enfin, une étape finale d'équeutage réalisée par distillation sépare la fraction lourde comprenant les sous-produits à haut point d'ébullition et les adduits de Michael, utilisable comme acide acrylique de grade ester brut.

En alternative, l'acide acrylique de grade ester brut peut être obtenu au cours de la purification d'acide acrylique récupéré à l'aide d'une colonne de déshydratation sans utiliser de solvant d'extraction ou de distillation azéotropique, en sortie du réacteur de synthèse de l'acide acrylique, comme décrit dans le brevet EP 2 066 613. Dans ce type de procédé, l'acide acrylique contenu dans le gaz issu de la section réaction est absorbé dans une première colonne à contre-courant d'un flux liquide essentiellement aqueux issu du gaz réactionnel, partiellement condensé et reflué en tête de colonne. Le flux concentré d'acide acrylique récupéré en fond de colonne est purifié dans une deuxième colonne qui réalise un étêtage complémentaire (élimination des légers résiduels en tête recyclés dans la première colonne) et un équeutage (récupération d'un acide acrylique de grade ester brut en pied), l'acide acrylique purifié de qualité technique étant soutiré latéralement. Les étapes de purification sont sensiblement équivalentes à celles du procédé utilisant une adsorption dans l'eau puis un solvant azéotropique, l'étape d'étêtage des produits légers étant réalisée dans la première colonne en même temps que l'étape de déshydratation, et l'étape finale de séparation des composés lourds étant réalisée dans la deuxième colonne.

Selon un mode de réalisation, l'acide acrylique de grade ester brut comprend, ou est constitué de, la fraction lourde séparée en pied de la dernière étape de purification dénommée équeutage dans un procédé de synthèse d'acide acrylique.

Selon un mode de réalisation, ledit flux d'acide acrylique de grade ester brut comprend en partie le flux séparé en pied de l'étape d'équeutage dans un procédé de synthèse d'acide acrylique.

Les conditions opératoires du procédé selon l'invention sont adaptées de façon à dissocier quasi quantitativement les dérivés d'addition de Michael et oligomères présents dans ledit flux d'acide acrylique de grade ester brut pour régénérer le monomère acide acrylique, et effectuer la réaction d'estérification. Selon l'invention, on obtient un ester acrylique avec un rendement supérieur à 95%, exprimé en pourcentage molaire d'ester fabriqué par rapport à l'acide acrylique valorisable contenu dans l'acide acrylique de grade ester, essentiellement introduit sous la forme de monomère, dimère ou trimère :

$$\mathrm{Re}\,ndement = \frac{m_{ester}/MM_{ester}}{(m_{AA\,monomère} + m_{AA\,dimère} + m_{AA\,trimère})/72}$$

Avec $m_X$ = masse de l'espèce x et $MM_X$ = masse molaire de l'espèce X

Section de réaction et récupération de l'ester acrylique brut

La zone réactionnelle, comprenant en général un réacteur d'estérification raccordé à une unité de distillation, est alimentée en continu par le flux d'acide acrylique de grade ester brut, un alcool léger (méthanol ou éthanol), et un catalyseur d'estérification. Le débit moyen d'alimentation des réactifs est généralement compris entre 0,1 et 0,5 T/hr par $m^3$ de volume utile du réacteur, de préférence entre 0,2 et 0,3 T/hr par $m^3$ de volume utile du réacteur.

La réaction d'estérification est faite en présence d'un excès molaire d'alcool par rapport à l'acide acrylique présent sous forme de monomère, dimère et trimère.

Le rapport molaire alcool par rapport à l'acide acrylique présent sous forme de monomère ou d'oligomère est généralement compris entre 1,2 et 1,5, de préférence entre 1,3 et 1,45. L'alcool est introduit dans le réacteur, seul ou en mélange avec d'autres réactifs ou flux recyclés, de préférence directement dans la phase liquide constituée par le milieu réactionnel maintenu sous agitation efficace, ou à travers des systèmes de distribution statiques ou dynamiques,

comme par exemple en amont d'une pompe, qui permettent la dispersion du réactif sous forme de fines gouttelettes. Les systèmes connus de mélangeurs permettant le mélange rapide de 2 liquides ou la dispersion rapide d'un gaz dans un liquide peuvent être utilisés.

La réaction proprement dite d'estérification s'effectue dans le réacteur à une température élevée de façon à dissocier simultanément les adduits de Michael, généralement à une température supérieure à 130°C, de préférence allant de 135°C à 155°C, voire de 140°C à 145°C, sous pression allant généralement de 0,9 à 1,3 bars.

Comme catalyseur d'estérification, on utilise généralement un acide fort minéral, comme l'acide sulfurique ou l'acide phosphorique, ou un acide organique, tel que l'acide méthane sulfonique (AMS), l'acide para-toluène sulfonique, l'acide benzène sulfonique, l'acide dodécylbenzenesulfonique, l'acide xylène sulfonique, ou leurs mélanges. De préférence, on utilise l'acide méthane sulfonique comme catalyseur d'estérification.

Le catalyseur est avantageusement introduit de façon continue afin de maintenir une concentration dans le réacteur supérieure à 2,5%, de préférence allant de 3% à 5% par rapport au milieu réactionnel.

Pour limiter la formation de polymères pendant la réaction, un (ou des) inhibiteur(s) de polymérisation est (ou sont) introduit(s) en même temps que le flux d'alimentation en réactifs. Comme exemples d'inhibiteurs de polymérisation, on peut utiliser la phénothiazine, ou un dérivé de la phénothiazine, des dérivés aminés tels que diphényl amine, ou diphénylène amine, des composés phénoliques tels que hydroquinone, éther monométhylique d'hydroquinone, diterbutyl para-crésol (BHT), ou ditertiobutylcatéchol, ou les composés N-oxyl tel que le TEMPO (2,2,6,6-tétraméthyl-1-piperidinyloxy) ou les dérivés du TEMPO, tel que le 4-hydroxy-TEMPO, des sels de manganèse, ou des composés de cuivre tels que des carbamates de cuivre, seuls ou leurs mélanges en toutes proportions.

De préférence, on utilise la phénothiazine ou un mélange de phénothiazine et d'hydroquinone comme inhibiteur de polymérisation.

L'inhibiteur de polymérisation peut être introduit dans le réacteur et/ou en tête de l'unité de distillation dans la zone réactionnelle.

L'inhibiteur de polymérisation est introduit dans la zone réactionnelle de façon à ce que la concentration en inhibiteur de polymérisation dans le réacteur soit maintenue à une valeur supérieure à 50 ppm, de préférence supérieure à 100 ppm, plus préférentiellement à une valeur comprise entre 300 ppm et 1000 ppm. La concentration en inhibiteur peut être contrôlée par analyse, par exemple par chromatographie liquide.

Ce niveau de concentration permet, d'une part de s'affranchir de la perte éventuelle d'efficacité de l'inhibiteur de polymérisation, d'autre part de maintenir une efficacité suffisante du catalyseur, en raison de ses réactions possibles avec le catalyseur acide, et en outre de réduire la viscosité du résidu final de réaction dans le réacteur.

Il peut être avantageux d'ajouter la phénothiazine comme inhibiteur de polymérisation à différents endroits de la zone réactionnelle, par exemple dans le réacteur, dans l'unité de distillation, et au niveau du reflux en tête de l'unité de distillation.

Il peut être avantageux d'introduire dans la zone réactionnelle un gaz contenant de l'oxygène, en particulier lorsque l'inhibiteur de polymérisation contient un composé phénolique.

La réaction est conduite dans le réacteur pendant une durée telle que la récupération des produits valorisables générés par la dissociation thermique et le rendement d'estérification soient très élevés, et telle que la quantité de résidu de réaction à éliminer soit très faible.

Cela correspond à un temps de séjour du résidu de réaction dans le réacteur supérieur à 50 heures, de préférence supérieur à 100 heures, le temps de séjour étant exprimé par la durée moyenne pendant laquelle le résidu de réaction est maintenu dans le réacteur avant d'être purgé, calculé par le ratio du volume de milieu réactionnel sur le débit de purge. Dans les conditions de la réaction, le résidu de réaction, enrichi en produits lourds provenant essentiellement du flux d'acide acrylique réactionnel, reste suffisamment fluide pour être soutiré à l'aide d'une pompe et pouvoir être envoyé à un oxydeur thermique pour élimination et récupération éventuelle d'énergie, ou à tout autre équipement en vue d'une valorisation ultime.

Préférentiellement, la viscosité dynamique du résidu mesurée à 100°C doit être inférieure à 200 cP, mesurée à l'aide d'un viscosimètre, comme par exemple un viscosimètre rotatif de Brookfield. Il peut être avantageux de lui ajouter un agent abaisseur de viscosité, par exemple du méthanol ou de l'acide acétique, à une teneur allant de 10 à 30%, pour faciliter les opérations ultérieures de pompage après stockage ou transport à plus faible température en vue de son élimination ou son recyclage comme fluide calorifique par exemple.

De manière avantageuse, le résidu de réaction est prélevé à la température de la réaction d'estérification permettant la dissociation thermique des adduits de Michael et il est envoyé dans un stockage, maintenu à une température proche de celle de la réaction à moins de 10°C près Les évents gazeux du bac de stockage sont collectés et recyclés vers le réacteur, permettant ainsi, durant cette phase de stockage avant élimination, une récupération supplémentaire d'ester acrylique à partir du résidu.

Les étapes de réaction (estérification et craquage) et récupération (élimination et purification des produits de la réaction) sont étroitement liées. Les conditions de réaction contrôlent la composition du mélange de réaction dans le réacteur, qui conditionne la formation de mélanges azéotropiques acrylate léger / eau et acrylate léger / alcool léger.

Ces mélanges sont purifiés dans l'unité de distillation et dépendent également de la composition du reflux imposé en tête de ladite unité. En retour, les mélanges formés ont un impact important sur l'efficacité de la réaction attendue.

Pour exemple, lors de l'estérification de l'acide acrylique par le méthanol pour produire l'acrylate de méthyle, coexistent un premier mélange azéotropique acrylate de méthyle / méthanol relativement riche en méthanol (52% en théorie) et un second mélange azéotropique acrylate de méthyle / eau. Le premier mélange azéotropique a un impact négatif sur l'avancement de la réaction en favorisant l'élimination d'un réactif (l'alcool) du milieu réactionnel. Le second mélange azéotropique a un effet positif puisqu'il permet de déplacer l'équilibre d'estérification, mais son élimination est défavorisée par rapport au premier mélange en raison d'un point d'ébullition supérieur de 9°C et du fait de sa relative pauvreté en eau (9% en théorie).

Par conséquent, les conditions de réaction tendant à favoriser la formation du second mélange azéotropique (acrylate de méthyle / eau) plutôt que le premier (acrylate de méthyle / méthanol) permettent d'améliorer le rendement de la réaction.

Elles sont réalisées dans une zone réactionnelle comprenant un réacteur et une unité de distillation permettant l'élimination simultanée de l'eau produite par la réaction d'estérification, de l'ester fabriqué, de l'alcool excédentaire qui n'a pas réagi, ainsi que de faibles quantités d'impuretés résiduelles ou générées pendant la réaction pour former un mélange appelé ester brut.

La zone réactionnelle peut être un réacteur dont la phase gaz est connectée avec une colonne de distillation, ou une colonne réactive constituée en partie basse d'une section réaction contenant le milieu réactionnel liquide et en partie haute d'une section de distillation.

Le réacteur peut être tout type de réacteur agité connu de l'homme de l'art. De manière préférentielle, le réacteur ou la section réaction de la colonne réactive sont alimentés en continu par le mélange de réactifs, une partie du mélange réactionnel est prélevée et réchauffée dans un échangeur externe et le flux réchauffé est recyclé dans le réacteur à l'aide d'une pompe.

De manière préférentielle, le réacteur, l'échangeur, la pompe, les conduites de transfert et tout équipement en contact avec le milieu réactionnel sont en matériau résistant à la corrosion ou sont revêtus de matériaux résistant à la corrosion.

La colonne de distillation ou la section distillation de la colonne réactive peut être constituée de tout type de plateaux et/ou d'internes vrac et/ou de garnissages structurés disponibles pour la rectification de mélanges et adaptés à la distillation de composés polymérisables. Elle est équipée d'un condenseur et d'une alimentation liquide en tête, qui assure un reflux liquide dans la colonne.

Le nombre de plateaux et / ou la hauteur et le type de garnissage de la colonne sont choisis de façon à limiter l'entrainement d'acide acrylique n'ayant pas réagi dans l'effluent récupéré en tête de colonne.

De manière optionnelle, en aval du condenseur, un décanteur peut être installé pour séparer une phase organique contenant l'essentiel de l'ester et des traces d'eau et d'alcool n'ayant pas réagi, et une phase aqueuse contenant l'essentiel de l'eau générée par la réaction et de l'alcool qui n'a pas réagi, ainsi que de faibles quantités d'ester

L'élimination de l'eau générée par l'estérification est réalisée essentiellement par entrainement sous forme de mélange azéotropique avec l'ester léger fabriqué. Afin de limiter l'entrainement de l'alcool léger non converti par le mélange azéotropique avec l'ester fabriqué, qui aurait pour conséquence une diminution du rendement de la réaction, un excès d'ester est maintenu de façon à favoriser la réaction d'estérification par élimination de l'eau formée. Ceci est réalisé grâce à un reflux d'une phase d'acrylate léger essentiellement exempt d'eau qui, exprimé en débit massique par rapport au débit massique de l'alimentation des réactifs dans la zone réactionnelle, est maintenu supérieur à 0,8 , de préférence entre 1 et 2,5, voire entre 1 et 1,2.

Ledit acrylate léger reflué exempt d'eau, contenant de préférence moins de 5% massique d'eau, peut provenir d'une partie de la phase organique séparée dans le décanteur et / ou d'une fraction séparée lors de la purification de l'effluent distillé, par exemple en pied d'une colonne de distillation des composés légers ou en pied d'une colonne de séparation des composés lourds et permet ainsi de recycler l'acrylate léger présent dans ces fractions.

L'effluent distillé comprenant le mélange d'ester brut, est soumis, soit après décantation, soit directement, à une chaine de traitement et de purification conduisant à l'obtention d'un acrylate léger purifié.

Selon le procédé de l'invention, l'acrylate léger contenu dans le mélange d'ester brut est produit avec un rendement supérieur à 95%, généralement allant de 95% à 98%, exprimé en nombre de moles d'acrylate léger produites par rapport au nombre de moles d'acide acrylique introduites sous forme d'acide acrylique monomère, dimère ou trimère.

Section de purification

Chacune des phases (organique et aqueuse) obtenues par décantation de l'ester brut subit un traitement de purification, visant à récupérer l'ester purifié contenu essentiellement dans la phase organique en éliminant l'eau et les impuretés contenues en faible concentration et en récupérant l'alcool qui s'y trouve, et à récupérer à des fins de recyclage l'alcool et les faibles concentrations d'ester qui sont présentes dans la phase aqueuse.

De façon avantageuse, ceci est réalisé dans une étape d'extraction liquide - liquide appliquée au mélange d'ester brut après décantation, de façon à augmenter la concentration d'alcool dans la phase aqueuse et réduire cette concentration dans la phase organique, et ainsi améliorer la récupération de l'alcool à des fins de recyclage à l'étape de réaction.

La colonne d'extraction est alimentée en pied par la phase organique issue de la décantation et en tête par le flux aqueux récupéré en pied de la colonne de récupération d'alcool léger.

La phase aqueuse obtenue en pied de la colonne d'extraction, enrichie en alcool, est avantageusement envoyée en partie sur une colonne de distillation pour récupérer en tête l'alcool léger qui est recyclé ensuite à la réaction, et en pied une phase aqueuse appauvrie en alcool léger pouvant être utilisée comme solvant d'extraction alimenté en tête de la colonne d'extraction. Une partie de cette phase aqueuse est éliminée.

En alternative, l'effluent d'ester brut distillé à partir de la zone réactionnelle peut être envoyé directement au niveau de la colonne d'extraction, sans séparation préalable dans un décanteur, minimisant ainsi l'appareillage nécessaire pour le traitement de l'effluent distillé, et facilitant le contrôle des opérations de séparation et recyclage de l'alcool résiduel et/ou de l'eau de lavage.

La phase organique lavée, est ainsi débarrassée de l'essentiel de l'alcool léger et comprend l'acrylate léger recherché, mais contient encore comme impuretés, des sous-produits légers et des sous-produits lourds.

La phase organique lavée est envoyée sur une première colonne de distillation permettant de débarrasser l'acrylate léger des sous-produits légers qu'il contient, dont notamment des traces d'alcool, acétates, diméthyléther ou diéthyléther ; ceux-ci sont soutirés en tête de ladite colonne pour être, en partie recyclés dans la zone réactionnelle ou à l'étape d'extraction, et en partie éliminés.

En pied de ladite colonne de distillation, on récupère l'acrylate léger comportant encore des impuretés lourdes dont notamment des alkoxy propionate de méthyle ou d'éthyle, de faibles quantités d'acryloxy propionates de méthyle ou d'éthyle, de maléate de diméthyle ou de diéthyle et de benzoate de méthyle ou d'éthyle et des inhibiteurs de polymérisation.

Ce flux est envoyé sur une colonne de séparation pour une purification finale. En pied de la colonne de séparation, on récupère un acrylate léger concentré en impuretés lourdes, qui est en partie éliminé, en partie recyclé comme reflux au niveau de l'unité de distillation dans la zone réactionnelle.

En tête de la colonne de séparation, on récupère un acrylate léger de pureté supérieure à 99%.

Les avantages de l'invention sont maintenant illustrés de façon non limitative dans les exemples suivants.

## EXEMPLES

### Exemple 1

Le montage expérimental est constitué d'un réacteur agité de volume utile 1 litre chauffé par recirculation dans sa double enveloppe d'huile chaude à température régulée, surmonté d'une colonne de distillation. Le réacteur est équipé d'une entrée pour l'alimentation via une pompe du mélange de réactifs, d'une alimentation séparée de catalyseur AMS 70% dans l'eau via une deuxième pompe, d'une mesure de température dans le liquide, d'un point de soutirage en fond. La colonne est équipée de 7 plateaux perforés à déversoirs, d'une entrée en tête de colonne pour l'alimentation du reflux via une troisième pompe, d'un condenseur vertical placé sur la phase gaz de sortant en tête de colonne, alimenté via une quatrième pompe par un mélange d'eau contenant de l'hydroquinone à 2%, d'un bac intermédiaire équipé d'un contrôle de niveau et d'un bac de réception décanteur soutirant à l'aide d'une cinquième pompe le mélange brut d'ester distillé.

Dans une première phase durant 3 semaines pendant laquelle les conditions opératoires et la composition du milieu évoluent, le mélange de résidu riche en composés lourds est constitué par enrichissement progressif du milieu réactionnel, pour atteindre finalement les conditions permettant de réaliser à la fois l'estérification de l'acide acrylique et le craquage thermique des oligomères présents dans l'acide acrylique réactif et générés pendant cette opération.

A l'issue de cette première phase d'enrichissement, les conditions opératoires et compositions sont stabilisées, la concentration d'AMS mesurée dans le milieu réactionnel est de 4.5%.

Un mélange d'alimentation, constitué de 57.9% d'acide acrylique de grade ester brut, de 32.4% méthanol, et 7.6% d'acrylate de méthyle et 2.1% d'eau (ces 2 composés étant issus du recyclage de flux provenant d'étapes ultérieures) est alimenté dans le réacteur avec un débit de 300g/h. L'acide acrylique de qualité ester brut est composé de 84.4% d'acide acrylique, 12.8% d'acide acrylique dimère et 0.6% d'acide acrylique trimère, 0.5% de phénothiazine, 0.3% d'hydroquinone et 1.5% d'autres composés. Le catalyseur AMS 70% dans l'eau est ajouté avec un débit de 0.97g/h. En tête de colonne de distillation, de l'acrylate de méthyle pur contenant 0.1% de phénothiazine est envoyé en reflux avec un débit de 330g/h.

La réaction est conduite pendant 196h à une température de 140°C dans ces conditions, avec les paramètres opératoires suivants :

- le rapport molaire d'alcool / acide acrylique valorisable (somme d'acide acrylique monomère, dimère et trimère) est de 1.3,
- le débit d'alimentation par unité de volume réactionnel utile de 0.3T/h/$m^3$,
- le ratio de débit de reflux / alimentation des réactifs est de 1.1,
- le temps de séjour moyen du résidu dans le réacteur, calculé par le ratio du volume de réacteur occupé sur le débit de purge de résidu, est de 116h
- la concentration d'AMS présent dans le milieu réactionnel est de 4.5%, déterminée par mesure de l'acidité,
- la concentration de phénothiazine mesurée par analyse dans le réacteur est de 0.05%

Le mélange d'ester brut condensé en tête de colonne décante en 2 phases qui sont séparées et analyses séparément. Sur une période de prélèvement de 16h, on obtient 9532g de phase organique composée de 3.2% de méthanol, 5.15% d'eau, 0.26% d'acide acrylique, le restant étant essentiellement de l'acrylate de méthyle et 458g de phase aqueuse constituée de 13.1% de méthanol; 7.33% d'acrylate de méthyle et 0.06% d'acide acrylique, le reste étant essentiellement constitué d'eau.

Le rendement de la réaction, déterminé par le ratio molaire d'acrylate de méthyle produit (soustraction faite de l'acrylate de méthyle alimenté par le reflux et le flux d'alimentation) par rapport à l'acide acrylique valorisable alimenté (somme d'acide acrylique monomère, dimère et trimère) est de 95.2%. Ce rendement est également le rendement moyen obtenu durant 1 semaine de fonctionnement.

La viscosité dynamique du résidu réactionnel, mesurée à l'aide d'un viscosimètre BROOKFIELD CAP1000+ à une température de 100°C, est de 150cP.

<u>Exemple 2</u>

Le réacteur est opéré de la même façon que lors de l'essai 1, hormis les changements suivants :

- température de réaction : 143°C,
- rapport molaire d'alcool / acide acrylique valorisable (somme d'acide acrylique monomère, dimère et trimère) : 1.4
- concentration d'AMS : 3.3%
- mélange envoyé en reflux en tête de colonne, constitué par de l'acrylate de méthyle contenant 0.5% de méthanol, 2.6% d'acétate de méthyle, 2.4% d'eau, de façon à prendre en considération le recyclage d'un mélange issu des étapes suivantes du procédé, contenant quelques impuretés

Dans ces conditions dégradées par le recyclage d'impuretés dans le mélange de reflux, maintenues constantes pendant 200h, le temps de séjour moyen du résidu dans le réacteur est de 114h et le rendement moyen de la réaction sur une période de 100h de fonctionnement atteint 97.8%.

La viscosité dynamique du résidu réactionnel, mesurée à une température de 100°C, est de 160cP, avec une concentration de phénothiazine mesurée de 0.05%.

<u>Exemple 3 (comparatif)</u>

Le réacteur est opéré durant 53h de la même façon que lors de l'essai 1, avec les paramètres opérationnels suivants :

- Composition de l'acide acrylique de grade ester brut : 88.8% d'acide acrylique monomère, 9% d'acide acrylique dimère, 0.3% d'acide acrylique trimère, 0.27% de phénothiazine, 0.19% d'hydroquinone,
- débit d'alimentation par unité de volume réactionnel utile de 0.3T/h/$m^3$,
- rapport molaire d'alcool / acide acrylique valorisable (somme d'acide acrylique monomère, dimère et trimère) est de 1.3
- ratio de débit de reflux / débit d'alimentation des réactifs de 1.1,
- concentration d'AMS : 11%
- temps de séjour du résidu supérieur à 300h

La température de réaction est réduite à 128°C, et la viscosité du résidu mesurée à 100°C est de 150 cP
Malgré la forte concentration de catalyseur mise en jeu, le rendement moyen calculé durant l'opération n'est que de 92.7%

<u>Exemple 4 (comparatif)</u>

Le réacteur est opéré durant 47h de la même façon, avec le même flux d'alimentation et dans les mêmes

conditions que l'essai 3, hormis :

- un rapport molaire d'alcool / acide acrylique valorisable (somme d'acide acrylique monomère, dimère et trimère) est de 1.45
- une concentration d'AMS de 10%
- une température de réaction de 135°C

Grâce à la température de réaction supérieure à celle de l'essai 3, le rendement moyen calculé durant l'opération est de 97%. En revanche, la viscosité du milieu réactionnel mesurée à 100°C est bien supérieure à 250 cP (limite de mesure du viscosimètre), rendant très difficile la vidange du réacteur, et des solides ont pu être observés durant cette opération de vidange. La concentration de phénothiazine mesurée dans le milieu réactionnel est inférieure à 10 ppm.

<u>Exemple 5 (comparatif)</u>

Le réacteur est opéré durant 62h de la même façon que lors de l'essai 1, avec les paramètres opérationnels suivants :

- Composition de l'acide acrylique de grade ester brut : 75.5% d'acide acrylique monomère, 18.8% d'acide acrylique dimère, 1% d'acide acrylique trimère, 0.85% de phénothiazine, 0.42% d'hydroquinone,
- débit d'alimentation par unité de volume réactionnel utile de 0.3T/h/$m^3$,
- rapport molaire d'alcool / acide acrylique valorisable (somme d'acide acrylique monomère, dimère et trimère) est de 1.3
- ratio de débit de reflux / débit d'alimentation de 1.1,
- température de réaction : 140°C,

La concentration d'AMS dans le milieu réactionnel est réduite à 2.2%

Le rendement moyen calculé durant l'opération n'atteint que 85.9%. En raison de la faible réactivité de la réaction, le débit de purge pour assurer un niveau constant dans le réacteur est augmenté, et le temps de séjour qui découle de ces conditions dégradées est de 34h.

<u>Exemple 6 (comparatif)</u>

Le réacteur est opéré durant 46h de la même façon que lors de l'essai 1, avec les paramètres opérationnels suivants :

- Composition de l'acide acrylique de grade ester brut : 88.8% d'acide acrylique monomère, 9% d'acide acrylique dimère, 0.3% d'acide acrylique trimère, 0.27% de phénothiazine, 0.19% d'hydroquinone,
- débit d'alimentation par unité de volume réactionnel utile de 0.3T/h/$m^3$,
- ratio de débit de reflux / débit d'alimentation de 1.1,
- température de réaction : 140°C,
- concentration d'AMS : 4%

Le rapport molaire d'alcool / acide acrylique valorisable (somme d'acide acrylique monomère, dimère et trimère) est réduit à 1.1.

Le temps de séjour moyen du résidu dans le réacteur est de 87h et le rendement moyen calculé durant l'opération n'est que de 87.5%.

## Revendications

1. Procédé de préparation en continu d'acrylate léger choisi parmi l'acrylate de méthyle et l'acrylate d'éthyle par réaction de l'alcool léger correspondant choisi parmi le méthanol et l'éthanol avec un flux d'acide acrylique de grade ester brut, en présence d'au moins un catalyseur acide et d'au moins un inhibiteur de polymérisation, dans une zone réactionnelle comprenant un réacteur raccordé à une unité de distillation, **caractérisé en ce que** :

   - le flux d'acide acrylique de grade ester brut comprend des oligomères d'acide acrylique à une teneur massique supérieure à 8% ;
   - le rapport molaire d'alcool par rapport à l'acide acrylique contenu sous forme de monomère, dimère ou trimère

dans le flux d'acide acrylique de grade ester brut est compris entre 1,2 et 1,5, de préférence entre 1,3 et 1,45 ;
- la température du réacteur est supérieure à 130°C, de préférence comprise entre 135°C et 155°C ;
- la concentration massique de catalyseur acide est maintenue supérieure à 2,5 %, de préférence comprise entre 3% et 5% dans le milieu réactionnel ;
- on ajuste la concentration en inhibiteur de polymérisation dans le réacteur à une valeur supérieure à 50 ppm, de préférence supérieure à 100 ppm ;
- on maintient un excès d'acrylate léger formé dans le réacteur ;
- l'effluent sortant de l'unité de distillation est soumis à une chaîne de traitement et de purification conduisant à l'obtention d'un acrylate léger purifié ;
- on maintient un temps de séjour du résidu de réaction dans le réacteur supérieur à 50 heures, de préférence supérieur à 100 heures.

**2.** Procédé selon la revendication 1 **caractérisé en ce que** l'acrylate léger est l'acrylate de méthyle.

**3.** Procédé selon la revendication 1 ou 2 caractérisé en que l'acide acrylique provient d'un procédé de production utilisant le propylène comme matière première.

**4.** Procédé selon la revendication 1 ou 2 caractérisé en que l'acide acrylique provient d'un procédé de production utilisant le glycérol ou la glycérine comme matière première, ou d'un procédé de déshydratation de l'acide lactique, de l'acide 3-hydroxypropionique ou de leurs sels d'ammonium..

**5.** Procédé selon l'une quelconque des revendications précédentes **caractérisé en ce que** le flux d'acide acrylique de grade ester brut est obtenu au cours de la purification d'acide acrylique brut récupéré à l'aide d'une colonne d'adsorption alimenté en un solvant, tel que l'eau ou un solvant hydrophobe, en sortie du réacteur de synthèse de l'acide acrylique.

**6.** Procédé selon l'une quelconque des revendications 1 à 4 **caractérisé en ce que** le flux d'acide acrylique de grade ester brut est obtenu au cours de la purification d'acide acrylique récupéré à l'aide d'une colonne de déshydratation sans utiliser de solvant d'extraction ou de distillation azéotropique, en sortie du réacteur de synthèse de l'acide acrylique.

**7.** Procédé selon l'une quelconque des revendications précédentes **caractérisé en ce que** le flux d'acide acrylique de grade ester brut comprend, ou est constitué de, la fraction lourde séparée en pied de la dernière étape de purification dénommée équeutage dans un procédé de synthèse d'acide acrylique.

**8.** Procédé selon l'une quelconque des revendications précédentes **caractérisé en ce que** l'on envoie en reflux dans la zone réactionnelle une phase d'acrylate léger contenant moins de 5% massique d'eau, avec un débit massique supérieur à 0,8, de préférence compris entre 1 et 2,5, exprimé par rapport au débit massique d'alimentation des réactifs.

**9.** Procédé selon l'une quelconque des revendications précédentes **caractérisé en ce que** le catalyseur est l'acide méthane sulfonique.

**10.** Procédé selon l'une quelconque des revendications précédentes **caractérisé en ce que** l'on utilise la phénothiazine ou un mélange de phénothiazine et d'hydroquinone comme inhibiteur de polymérisation.

**11.** Procédé selon l'une quelconque des revendications précédentes **caractérisé en ce que** la viscosité dynamique du résidu de réaction est inférieure à 200 cP, mesurée à 100°C à l'aide d'un viscosimètre rotatif de Brookfield.

## Patentansprüche

**1.** Verfahren zur kontinuierlichen Herstellung von leichtem Acrylat, das aus Methylacrylat und Ethylacrylat ausgewählt wird, durch Umsetzung des entsprechenden leichten Alkohols, der aus Methanol und Ethanol ausgewählt wird, mit einem Strom von Acrylsäure mit Rohesterqualität in Gegenwart von mindestens einem sauren Katalysator und mindestens einem Polymerisationsinhibitor in einer Reaktionszone, die einen an eine Destillationseinheit angeschlossenen Reaktor umfasst, **dadurch gekennzeichnet, dass**

- der Strom von Acrylsäure mit Rohesterqualität Acrylsäureoligomere in einem Massengehalt von mehr als 8 % umfasst;
- das Molverhältnis von Alkohol zu Acrylsäure, die in Monomer-, Dimer- oder Trimer-Form in dem Strom von Acrylsäure mit Rohesterqualität enthalten ist, zwischen 1,2 und 1,5 und vorzugsweise zwischen 1,3 und 1,45 liegt;
- die Reaktortemperatur über 130 °C und vorzugsweise zwischen 135 °C und 155 °C liegt;
- die Massenkonzentration an saurem Katalysator im Reaktionsmedium über 2,5 % und vorzugsweise zwischen 3 % und 5 % gehalten wird;
- die Konzentration an Polymerisationsinhibitor im Reaktor auf einen Wert über 50 ppm und vorzugsweise über 100 ppm eingestellt wird;
- ein Überschuss von im Reaktor gebildetem leichtem Acrylat aufrechterhalten wird;
- der aus der Destillationseinheit austretende Austragsstrom einer Behandlungs- und Reinigungskette unterworfen wird, die zum Erhalt eines gereinigten leichten Acrylats führt;
- eine Verweilzeit des Reaktionsrückstands im Reaktor von mehr als 50 Stunden und vorzugsweise mehr als 100 Stunden aufrechterhalten wird.

**2.** Verfahren nach Anspruch 1, **dadurch gekennzeichnet, dass** es sich bei dem leichten Acrylat um Methylacrylat handelt.

**3.** Verfahren nach Anspruch 1 oder 2, **dadurch gekennzeichnet, dass** die Acrylsäure aus einem Herstellungsverfahren, bei dem Propylen als Ausgangsstoff verwendet wird, stammt.

**4.** Verfahren nach Anspruch 1 oder 2, **dadurch gekennzeichnet, dass** die Acrylsäure aus einem Herstellungsverfahren, bei dem Glycerol oder Glycerin als Ausgangsstoff verwendet wird, oder einem Verfahren zur Dehydratisierung von Milchsäure, 3-Hydroxypropionsäure oder den Ammoniumsalzen davon stammt.

**5.** Verfahren einem der vorhergehenden Ansprüche, **dadurch gekennzeichnet, dass** der Strom von Acrylsäure mit Rohesterqualität im Lauf der Reinigung von mit Hilfe einer mit einem Lösungsmittel, wie Wasser oder einem hydrophoben Lösungsmittel, gespeisten Adsorptionskolonne am Ausgang des Acrylsäure-Synthesereaktors gewonnener Rohacrylsäure erhalten wird.

**6.** Verfahren nach einem der Ansprüche 1 bis 4, **dadurch gekennzeichnet, dass** der Strom von Acrylsäure mit Rohesterqualität im Lauf der Reinigung von mit Hilfe einer Dehydratisierungskolonne ohne Verwendung von Extraktionslösungsmittel oder azeotroper Destillation am Ausgang des Acrylsäure-Synthesereaktors gewonnener Acrylsäure erhalten wird.

**7.** Verfahren nach einem der vorhergehenden Ansprüche, **dadurch gekennzeichnet, dass** der Strom von Acrylsäure mit Rohesterqualität die am Sumpf des als Tailing bezeichneten letzten Reinigungsschritts bei einem Verfahren zur Synthese von Acrylsäure abgetrennte schwere Fraktion umfasst oder daraus besteht.

**8.** Verfahren nach einem der vorhergehenden Ansprüche, **dadurch gekennzeichnet, dass** man eine Phase von leichtem Acrylat, die weniger als 5 Massen-% Wasser enthält, mit einem Massendurchsatz von mehr als 0,8, vorzugsweise zwischen 1 und 2,5, ausgedrückt in Bezug auf den Massendurchsatz der Reaktantenzufuhr, als Rückfluss der Reaktionszone zuführt.

**9.** Verfahren nach einem der vorhergehenden Ansprüche, **dadurch gekennzeichnet, dass** es sich bei dem Katalysator um Methansulfonsäure handelt.

**10.** Verfahren nach einem der vorhergehenden Ansprüche, **dadurch gekennzeichnet, dass** als Polymerisationsinhibitor Phenothiazin oder eine Mischung von Phenothiazin und Hydrochinon verwendet wird.

**11.** Verfahren nach einem der vorhergehenden Ansprüche, **dadurch gekennzeichnet, dass** die bei 100 °C mit einem Brookfield-Rotationsviskosimeter gemessene dynamische Viskosität des Reaktionsrückstands weniger als 200 cP beträgt.

## Claims

1. Process for the continuous preparation of light acrylate chosen from methyl acrylate and ethyl acrylate by reaction of the corresponding light alcohol chosen from methanol and ethanol with a stream of crude ester-grade acrylic acid, in the presence of at least one acid catalyst and of at least one polymerization inhibitor, in a reaction zone comprising a reactor connected to a distillation unit, **characterized in that**:

    - the stream of crude ester-grade acrylic acid comprises acrylic acid oligomers at a content by weight of greater than 8%;
    - the molar ratio of alcohol with respect to the acrylic acid contained in the monomer, dimer or trimer form in the stream of crude ester-grade acrylic acid is between 1.2 and 1.5, preferably between 1.3 and 1.45;
    - the temperature of the reactor is greater than 130°C, preferably of between 135°C and 155°C;
    - the concentration by weight of acid catalyst is kept greater than 2.5%, preferably of between 3% and 5%, in the reaction medium;
    - the concentration of polymerization inhibitor in the reactor is adjusted to a value of greater than 50 ppm, preferably of greater than 100 ppm;
    - an excess of light acrylate formed in the reactor is maintained;
    - the effluent exiting from the distillation unit is subjected to a treatment and purification line resulting in a purified light acrylate being obtained;
    - a residence time of the reaction residue in the reactor of greater than 50 hours, preferably of greater than 100 hours, is maintained.

2. Process according to Claim 1, **characterized in that** the light acrylate is methyl acrylate.

3. Process according to Claim 1 or 2, **characterized in that** the acrylic acid originates from a production process using propylene as starting material.

4. Process according to Claim 1 or 2, **characterized in that** the acrylic acid originates from a production process using glycerol or glycerine as starting material or from a process for the dehydration of lactic acid, of 3-hydroxypropionic acid or of their ammonium salts.

5. Process according to any one of the preceding claims, **characterized in that** the stream of crude ester-grade acrylic acid is obtained during the purification of crude acrylic acid recovered using an adsorption column fed with a solvent, such as water or a hydrophobic solvent, at the outlet of the reactor for the synthesis of the acrylic acid.

6. Process according to any one of Claims 1 to 4, **characterized in that** the stream of crude ester-grade acrylic acid is obtained during the purification of acrylic acid recovered using a dehydration column, without using extraction solvent or azeotropic distillation, at the outlet of the reactor for the synthesis of the acrylic acid.

7. Process according to any one of the preceding claims, **characterized in that** the stream of crude ester-grade acrylic acid comprises or consists of the heavy fraction separated at the bottom of the final purification stage, known as tailing, in a process for the synthesis of acrylic acid.

8. Process according to any one of the preceding claims, **characterized in that** a light acrylate phase containing less than 5% by weight of water is sent as reflux into the reaction zone with a flow rate by weight of greater than 0.8, preferably of between 1 and 2.5, expressed with respect to the feed flow rate by weight of the reactants.

9. Process according to any one of the preceding claims, **characterized in that** the catalyst is methanesulfonic acid.

10. Process according to any one of the preceding claims, **characterized in that** phenothiazine or a mixture of phenothiazine and hydroquinone is used as polymerization inhibitor.

11. Process according to any one of the preceding claims, **characterized in that** the dynamic viscosity of the reaction residue is less than 200 cP, measured at 100°C using a Brookfield rotary viscometer.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 2066613 A
- EP 887334 A
- FR 1351243
- US 3868410 A
- EP 2727964 A
- WO 9101966 A
- WO 0078702 A
- US 20040236143 A
- WO 9852903 A
- WO 9852904 A
- US 2007280866 A